# EUROPEAN PATENT APPLICATION

(11) **EP 1 250 928 A1**
(43) Date of publication of application: **23.10.2002**
(21) Application number: 01946757.0
(22) Date of filing: 25.01.2001
(51) Int. Cl.: A61K 31/765, A61P 35/04, A23L 1/30

(54) **CANCER CELL IMPLANTATION INHIBITORS**

(30) Priority: 26.01.2000 JP 2000016525
(71) Applicant: AMATO PHARMACEUTICAL PRODUCTS, LTD., Fukuchiyama-shi, Kyoto 620-0932 (JP)
(72) Inventor: MURAYAMA, Chieko, Ebina-shi, Kanagawa 243-0431 (JP); NAGATO, Yasukazu, Atsugi-shi, Kanagawa 243-0122 (JP); MURAKAMI, Masahiro, Eru Arukasaru 703, Osaka-shi Osaka 547-0026 (JP)
(74) Representative: Sternagel, Fleischer, Godemeyer & Partner Patentanwälte
(86) International application number: JP0100482
(87) International publication number: WO01054705

(57) **Abstract**

The object of the present invention is to provide an agent for preventing the implantation of cancer cells and/or an agent for preventing the cancer recurrence, for example, an agent and food and drink for preventing the implantation and/or metastasis of cancer cells existing in the transplanted organ, the graft and/or the transplanted cells in a recipient. According to the present invention, there is provided an agent for preventing the implantation of cancer cells, an agent for preventing the cancer recurrence, and food and drink for preventing the implantation of cancer cells and/or preventing the cancer recurrence, which comprises, as an active ingredient, a mixture of cyclic and/or straight chain poly lactic acids having a condensation degree of 3 to 19.

## Description

### TECHNICAL FIELD

The present invention relates to an agent for preventing the implantation of cancer cells and/or an agent for preventing the cancer recurrence. More specifically, the present invention relates to an agent for preventing the implantation of cancer cells and/or an agent for preventing the cancer recurrence, and food and drink for preventing the implantation of cancer cells and/or preventing the cancer recurrence, which comprises, as an active ingredient, a mixture of poly lactic acids having a condensation degree of certain range.

### BACKGROUND ART

In these days when malignant tumor (cancer) is the primary cause of death, interest and need on chemotherapy substances useful for the prevention and/or therapy of cancers are increased. In the chemotherapy of cancers, it is important to achieve anti-tumor effect, and also to reduce side effects such as emesis, diarrhea, psilosis or leucopenia due to myelopathy, and to extend the life span.

From studies that have been made so far, it has been reported that a mixture of cyclic and/or straight chain poly L-lactic acids having a condensation degree of 3 to 19 is useful as an antineoplastic agent (Japanese Patent Application Laying-Open (Kokai) Nos. 9-227388 and 10-130153) and also as a QOL improving agent for cancer patients (Japanese Patent Application No. 11-39894 Specification; Bulletin of Japan Society of Clinical Oncology, Vol. 33, No. 3, p. 493).

Further, it has been suggested that the aforementioned mixture of poly lactic acids has an effect of inhibiting or delaying canceration of normal cells (Study of canceration inhibition effect on mouse spontaneous cancer by administration of cyclic poly lactic acids; Bulletin of Japan Society of Clinical Oncology, Vol. 33, No. 3, p. 196, 1998) and has an effect of degenerating transplanted tumor cells (Study of anti-tumor effect of cyclic poly lactic acids in cancer-bearing mouse, Jpn,J.Cancer.Res. 88, Suppl. 605, 1997), and that the metastasis of tumor cells is inhibited by administering a mixture of poly lactic acids after the transplantation of tumor cells (Study of effect of cyclic poly lactic acids on the growth of Lewis solid cancer and its metastasis into lung;; Bulletin of Japan Society of Clinical Oncology, Vol. 33, No. 3, p. 196, 1998). It has been revealed that these anti-tumor effects which have already been confirmed are expressed due to the fact that the mixture of poly lactic acids is involved in the inhibition of anaerobic glycolysis of tumor cells. However, an anti-tumor effect in the case when the administration of the mixture of poly lactic acids has been started prior to the transplantation of tumor cells, has not yet been reported.

On the other hand, in an organ transplantation where an organ is removed from an individual and is transplanted into the same or different individual so as to make the organ function in the transplanted individual, there is possibility that cancer cells may exit in the transplanted organ, the graft or the transplanted cells. In such a case, it is necessary to prevent carcinogenesis and/or metastasis in the recipient who is subjected to transplantation. If implantation and metastasis of cancer cells which are contained in the externally transplanted organ, the graft or the transplanted cells can be prevented by previously administering an agent to a patient (recipient) to be subjected to transplantation, such an agent can be extremely useful in transplantation, and therefore development of such an agent has been sought.

### DISCLOSURE OF THE INVENTION

The object of the present invention is to provide an agent for preventing the implantation of cancer cells and/or an agent for preventing the cancer recurrence (for example, an agent for preventing the implantation and/or metastasis of cancer cells existing in the transplanted organ, the graft and/or the transplanted cells in a recipient), and food and drink for preventing the implantation of cancer cells and/or preventing the cancer recurrence.

In order to study for the purpose of solving the aforementioned objects, the present inventors have transplanted cancer cells into mice which was administered with a mixture of cyclic and/or straight chain poly lactic acids having a condensation degree of 3 to 19, and have studied the implantation ratio of the tumor and the survival ratio of the mice after certain period. As a result, it has been found that mice administered with a mixture of poly lactic acids show lower implantation ratio of the transplanted cancer and improved survival ratio of cancer-transplanted mice as compared with mice fed with normal feed. Further, as a result of studying the degree of lung metastasis of the transplanted cancer cells, it has been found that mice administered with a mixture of poly lactic acids prior to the transplantation of cancer cells show lower degree of lung metastasis as compared with mice not administered with a mixture of poly lactic acids. The present invention has been completed on the basis of these findings.

Thus, according to the present invention, there is provided an agent for preventing the implantation of cancer cells which comprises, as an active ingredient, a mixture of cyclic and/or straight chain poly lactic acids having a condensation degree of 3 to 19.

According to another aspect of the present invention, there is provided an agent for preventing the cancer recurrence which comprises, as an active ingredient, a mixture of cyclic and/or straight chain poly lactic acids having a condensation degree of 3 to 19.

The agent of the present invention is used for, for example, preventing the implantation and/or metastasis of cancer cells existing in the transplanted organ, the graft and/or the transplanted cells in a recipient.

The administration of the agent of the present invention preferably starts prior to surgery of cancer or transplantation.

Preferably, the lactic acid that is a repeating unit in the poly lactic acid consists substantially of L-lactic acid.

Preferably, the mixture of cyclic and/or straight chain poly lactic acids having a condensation degree of 3 to 19 is a fraction obtained by condensing lactic acids by dehydration under an inactive atmosphere, subjecting the ethanol- and methanol-soluble fractions of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

Preferably, condensation by dehydration is performed by stepwise decompression and temperature rise under nitrogen gas atmosphere.

Preferably, reverse phase column chromatography is performed by ODS column chromatography.

According to another aspect of the present invention, there are provided food and drink for preventing the implantation of cancer cells and/or preventing the cancer recurrence, which comprises, as an active ingredient, a mixture of cyclic and/or straight chain poly lactic acids having a condensation degree of 3 to 19.

According to still another aspect of the present invention, there is provided the use of a mixture of cyclic and/or straight chain poly lactic acids having a condensation degree of 3 to 19 in the production of an agent for preventing the implantation of cancer cells and/or an agent for preventing the cancer recurrence, and food and drink for preventing the implantation of cancer cells and/or preventing the cancer recurrence.

According to a still further aspect of the present invention, there is provided a method for preventing the implantation of cancer cells and/or preventing the cancer recurrence, which comprises a step of administering a therapeutically and/or preventively effective amount of a mixture of cyclic and/or straight chain poly lactic acids having a condensation degree of 3 to 19 to mammals such as humans.

### BRIEF DESCRIPTION OF THE DRAWINGS

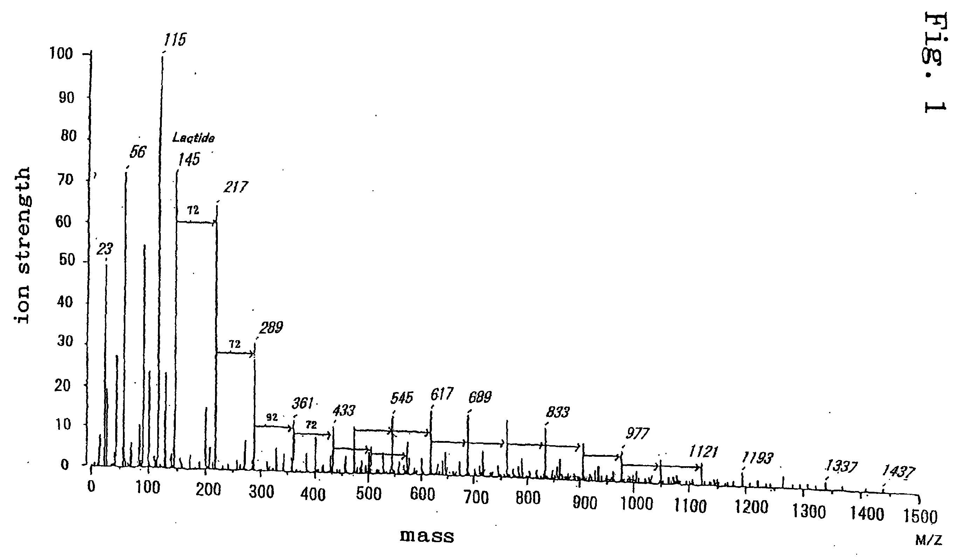

Figure 1 shows a mass spectrum of the mixture of poly lactic acids obtained by Production Example 1 of the present specification.

### THE BEST MODE FOR CARRYING OUT THE INVENTION

The embodiment and method for the practice of the present invention are described in detail below.

The agent of the present invention comprises, as an active ingredient, a mixture of cyclic and/or straight chain poly lactic acids having a condensation degree of 3 to 19, and is used for preventing the implantation of cancer cells and/or preventing the cancer recurrence, and more particularly for, for example, preventing recurrence after surgery of cancer or preventing the implantation and/or metastasis of cancer cells existing in the transplanted organ, the graft and/or the transplanted cells in a recipient.

The present inventors have found this time that the implantation (fixation) of externally implanted tumor cells can be inhibited and the survival ratio of cancer-bearing mice can be improved by administering a mixture of cyclic and/or straight chain poly lactic acids having a condensation degree of 3 to 19 to mice. These phenomena are novel knowledge which cannot be predicted from the conventional knowledge that a poly lactic acid mixture is involved in the inhibition of anaerobic glycolysis. Although the present invention is not restricted by the following theories, it is guessed that the poly lactic acid mixture is involved also in the regulation of the immune mechanism of a living body.

The term "implantation" used herein means that cancer cells proliferate autonomously at certain site, and includes that cancer cells existing in a patient metastasize to another site and proliferate autonomously there, and that cancer existing in the transplanted cells, the graft and/or the transplanted organ proliferate autonomously at the site where transplanted. The agent of the present invention can be used for preventing such an implantation of cancer cells. The agent of the present invention can be used also for preventing the cancer recurrence. The cancer recurrence refers to that, after certain period has passed after excision of cancer by surgery or the like, cancer starts implanting and proliferating at another site. The cancer recurrence occurs due to, for example, that the remaining cancer such as leftover cancer in the case of surgery metastasize to another site. By administering the agent of the present invention preferably prior to surgery, cancer recurrence can be prevented.

The term "transplantation" used herein means that cells, tissues or organs are infused or introduced within the same individual (autotransplantation), between different individuals (syngeneic, allogeneic transplantation), or between the individuals of different species (heterotransplantation). When the term "transplantation" is simply referred to, it includes all of autotransplantation, syngeneic/allogeneic transplantation and heterotransplantation. The cell transplantation includes blood infusion and bone marrow transplantation. The tissue transplantation includes corneal transplantation, bone transplantation, skin transplantation, vascular transplantation, a heart valve transplantation, and parathyroid transplantation. The organ transplantation includes kidney transplantation, liver transplantation, pancreas transplantation, lung transplantation, heart transplantation, and heart-and-lungs simultaneous transplantation. However, the examples are not limited to those mentioned above.

The term "metastasis" used herein means that cancer metastasizes. For example, it means that cancer existing in the transplanted cells, the graft or the transplanted organ metastasizes to another site. More particularly, it means that tumor cells are separated from transplanted cells, the graft or the transplanted organ, and are carried to a remote site (remote organ and the like), and proliferates autonomously there. The metastasis occurs mainly via 4 routes of lymphatic metastasis, hematogenous metastasis, contact metastasis and disseminative metastasis, but the routes are not limited thereto in the present invention.

The term "cancer" used herein has its broadest meaning which includes all the malignant tumors, and it includes all carcinoma (epithelial malignant tumor), sarcom (non-epithelial malignant tumor), and combined type thereof. The types of the transplanted cancer can also be classified according to its generating site. Examples thereof include hypophysis adenoma, glioma, acoustic neurilemoma, brain tumor, pharyngeal cancer, laryngeal cancer, thymoma, mesothelioma, breast cancer, lung cancer, stomach cancer, esophagus cancer, colon and rectum cancer, liver cell cancer, pancreatic cancer, pancreatic endocrine tumor, bile duct cancer, gallbladder cancer, penis cancer, ureter cancer, kidney cell cancer, a testis (spermary) tumor, prostatic cancer, bladder cancer, vulva cancer, uterine cancer, uterine sarcoma, vaginal cancer, breast cancer, ovarian cancer, ovarian embryo cell tumor, malignant melanoma, mycosis fungoides, skin cancer, soft tissue sarcoma, malignant lymphoma, a non-Hodgkin's lymphoma, myelodysplastic syndromes, multiple myeloma, plasma cell tumor, brown lymphoma, and pancreatic endocrine tumor, but the above are only examples of cancers and the types of the cancer are not limited thereto.

In the agent of the present invention, a mixture of cyclic and/or straight chain poly lactic acids having a condensation degree of 3 to 19 is used as an active ingredient.

The term "a mixture of poly lactic acids" used in the present invention means a mixture wherein cyclic and/or straight chain poly lactic acids having a condensation degree of 3 to 19 are present at any ratio. That is to say, the term "mixture" does not only mean a mixture of poly lactic acids having any condensation degree ranging from 3 to 19, but is also used as a concept including a mixture of cyclic and straight chain poly lactic acids. As is described below in the present specification, "a mixture of poly lactic acids" can be obtained by condensing lactic acids by dehydration and then performing purification by a suitable method. Although the term "a mixture of poly lactic acids" is used in the present specification for the sake of convenience, this term also includes a poly lactic acid consisting of a single ingredient such as a cyclic poly lactic acid having single condensation degree or a straight chain poly lactic acid having single condensation degree.

The term "condensation degree" is used to mean the number of lactic acid unit that is a repeating unit in poly lactic acids. For example, the cyclic poly lactic acid is assumed to have the following structural formula wherein n represents condensation degree (n = 3 to 19).

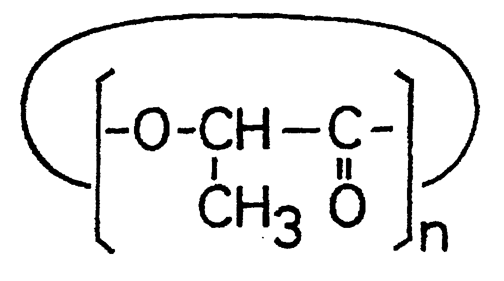

When "lactic acid" is simply referred to in the present specification, this lactic acid includes all of L-lactic acid, D-lactic acid or a mixture comprising these types of lactic acid at any ratio. Preferably in the present invention, the lactic acid consists substantially of L-lactic acid. The term "substantially" is used herein to mean that the ratio of L-lactic acid units in a mixture of poly lactic acids (number of L-lactic acid unit / number of L-lactic acid unit + number of D-lactic acid unit × 100) is, for example, 70% or more, preferably 80% or more, more preferably 85% or more, further more preferably 90% or more, and particularly preferably 95% or more. The ratio of L-lactic acid units in a mixture of poly lactic acids depends on the ratio of L-lactic acid and D-lactic acid that exist in lactic acids used as a starting substance.

The methods for producing a mixture of cyclic and/or straight chain poly lactic acids having a condensation degree of 3 to 19 are not particularly limited, and the mixture of poly lactic acids can be obtained by the production methods described, for example, in Japanese Patent Application Laying-Open (Kokai) Nos. 9-227388 and 10-130153 or Japanese Patent Application No. 11-39894 (All publications cited herein are incorporated herein by reference in their entirety).

More specifically, for example, a mixture of cyclic and/or straight chain poly lactic acids having a condensation degree of 3 to 19 can be obtained by the following method A.

### Method A:

First, lactic acid (preferably, lactic acid substantially consisting of L-lactic acid) is condensed by dehydration under an inactive atmosphere. Examples of the inactive atmosphere include nitrogen gas and argon gas, and nitrogen gas is preferred.

Dehydration and condensation reaction is carried out at a temperature of 110°C to 210°C, preferably 130°C to 190°C under normal pressure to reduced pressure of approximately 1mmHg, and particularly preferably the reaction is carried out by stepwise decompression and stepwise temperature rise. A reaction period can be determined as appropriate. For example, the reaction can be carried out for 1 to 20 hours. Where stepwise decompression and stepwise temperature rise are applied, reaction is performed by dividing the reaction period into two or more partial reaction periods, and then determining pressure and temperature for each of the reaction periods. Where stepwise decompression is applied, pressure can be reduced, for example, from a normal pressure to 150mmHg and then to 3mmHg. Where stepwise temperature rise is applied, temperature can be raised, for example, from 145°C to 155°C and then to 185°C. Practically, the reaction can be carried out by using these conditions in combination, for example, 145°C, normal pressure, 3 hours; 145°C, 150mmHg, 3 hours; 155°C, 3mmHg, 3 hours; and 185°C, 3mmHg, 1.5 hours.

Subsequently, ethanol and methanol are added to the reaction mixture obtained by the dehydration and condensation reaction, and the mixture is filtered. The obtained filtrate is dried to obtain ethanol- and methanol-soluble fractions. The term "ethanol- and methanol-soluble fractions" is used in the present specification to mean fractions soluble in a mixed solution of ethanol and methanol. In order to obtain ethanol and methanol-soluble fractions, a reaction mixture obtained by dehydration and condensation reaction is mixed with ethanol and methanol, where the ratio of ethanol and methanol can be determined as appropriate. For example, the ratio is ethanol:methanol = 1 : 9. The order, method and the like for adding ethanol and methanol to a reaction mixture are not limited, and may be selected as appropriate. For example, ethanol may be added at first to the reaction mixture obtained by the dehydration and condensation reaction, and then methanol may be added thereto.

The thus obtained ethanol- and methanol-soluble fractions are subjected to reverse phase column chromatography, especially to chromatography where an octadecylsilane (ODS) column is used. First, fractions eluted with 25 to 50 weight % acetonitrile aqueous solution of pH 2 to 3 are removed, and then fractions eluted with 90 weight % or more acetonitrile aqueous solution of pH 2 to 3, preferably 99 weight % or more acetonitrile aqueous solution, are collected so as to obtain a mixture of cyclic and/or straight chain poly lactic acids having a condensation degree of 3 to 19.

The thus obtained mixture of cyclic and/or straight chain poly lactic acids is neutralized with an alkaline substance such as sodium hydroxide, and is dried under reduced pressure, and then according to standard techniques, the mixture can be formulated in a desired form as mentioned below.

Other examples of the methods for producing a mixture of cyclic and/or straight chain poly lactic acids having a condensation degree of 3 to 19 used in the present invention include a method described in Japanese Patent Application No. 11-265715 (hereinafter referred to as method B), or a method described in Japanese Patent Application No. 11-265732 (hereinafter referred to as method C) (All publications cited herein are incorporated herein by reference in their entirety). Methods B and C will be described specifically below.

### Method B:

Method B is a method for producing a cyclic lactic acid oligomer which comprises polymerizing lactid in the presence of a lithium compound represented by RYLi [wherein R represents an aliphatic group or aromatic group, Y represents oxygen atom or sulfur atom]. In the case of performing the polymerization reaction, the ratio of the amounts of the lithium compound (RYLi) is 1-0.1 mol, preferably 0.2-0.3 mol per mol of lactide. The reaction temperature is -100 to 0°C, preferably -78 to -50°C. Reaction is preferably carried out by starting from a temperature of -78 to -50°C and gradually raising it to room temperature. The reaction is preferably carried out in the presence of a reaction solvent. As the reaction solvent, there can be used ,for example, a cyclic ether such as tetrahydrofuran, diethylether, and dimethoxyethane. The reaction atmosphere can be an inactive gas atmosphere such as nitrogen gas and argon. The reaction pressure is not limited, and is preferably a normal pressure.

The composition (that is, the mixing ratio of cyclic lactic acid oligomer and a chain lactic acid oligomer) of the lactic acid oligomer obtained as described above fluctuates depending on the lithium compound used as a reaction assistant. Where a lithium compound of alkyl alcohol having a carbon number of 1 to 3 (ROLi) (wherein R represents an alkyl group with carbon number 1 to 3) is used as a lithium compound, a mixture of a cyclic lactic acid oligomer and a chain oligomer (proportion of the cyclic lactic acid oligomer: 80 to 85 weight %) is obtained. When a lithium compound of alkyl alcohol having a carbon number of 4 or more such as t-butyl alcohol, or thiophenol compound is used as a lithium compound, substantially only a cyclic lactic acid oligomer can be selectively obtained.

### Method C:

This method comprises:
(i) a first heating step which comprises heating lactic acid under a pressure condition of 350 to 400 mmHg and to a temperature of 120 to 140°C so as to perform dehydration and condensation, and distilling off and removing only by-product water without distilling lactid off;
(ii) a second heating step for synthesizing a product condensed by dehydration comprising chain lactic acid oligomers as the main ingredient, which comprises, after completion of the first heating step, heating the reaction product to a temperature of 150 to 160°C while reducing the reaction pressure to 15 to 20 mmHg at a decompression rate of 0.5 to 1 mmHg/min, wherein only by-product water is distilled off and removed while avoiding distillation of lactid; and after the reaction pressure is reduced to 15 to 20 mmHg, maintaining the reaction under the same pressure condition and at a reaction temperature of 150 to 160°C;
(iii) a third heating step for synthesizing cyclic oligomers which comprises, after completion of the second heating step, heating under a pressure condition of 0.1 to 3 mmHg and at 150 to 160°C to cyclize the chain lactic oligomer.

In this method, first, in the first heating step, lactic acid is heated under reduced pressure to perform dehydration and compression reaction. In this case the reaction period is 3 to 12 hours, preferably 5 to 6 hours. To allow the reaction in the first heating step to proceed smoothly, by-product water produced by condensation of lactic acids by dehydration is distilled off. At this time, distillation of by-product water is performed such that lactid, which is the dehydrated condensed product of two molecules of lactic acid, is not distilled off. To achieve such purpose, the reaction pressure is maintained at a reduced pressure, preferably 300 to 500 mmHg, more preferably 350 to 400 mmHg. Under this pressure condition, heating is performed at a temperature range of 100 to 140°C, preferably 130 to 140°C. The reaction product produced by reaction in the first heating step mainly comprises as the main ingredient a dehydrated condensed product of 3 to 23 molecules of lactic acid.

To obtain oligomers having an increased average degree of polymerization in the second heating step after completion of the above first heating step, heating is performed at a temperature higher than the reaction temperature of the above first heating step, preferably at 145°C to 180°C, more preferably 150°C to 160°C, while the reaction pressure is reduced to 10 to 50 mmHg, preferably 15 to 20 mmHg, so that dehydration and condensation reaction is further continued.

As with the reaction in the above first heating step, reaction is performed under a condition where by-product water, but not lactid, is distilled off, to allow the reaction to proceed smoothly. The rate at which reaction pressure is reduced to a pressure in the above range (decompression rate) is normally required to be maintained within a range of 0.25 to 5 mmHg/min, preferably 0.5 to 1 mmHg/min, in order to avoid distillation of lactid and increase the reaction efficiency. A decompression rate lower than the above range is not preferred because it will increase the time required to reduce pressure to a given pressure. On the other hand, a decompression rate higher than the above range is also not preferred because it will cause lactid to be distilled off together with by-product water.

After the reaction pressure is reduced to a certain pressure, reaction is further continued at that reaction pressure. The heating time period in this case is 3 to 12 hours, preferably 5 to 6 hours.

A lactic acid oligomer having an average polymerization degree of 3 to 30, preferably 3 to 23 is obtained by the reaction in the above second heating step. The proportion of cyclic oligomers in the oligomers in this case is normally about 70 to 80 weight %.

In the third heating step, after completion of the above second heating step, a reaction pressure is maintained at 0.25 to 5 mmHg, preferably 0.5 to 1 mmHg, and reaction is further continued at a temperature of 145 to 180°C, preferably 150 to 160°C. A reaction period is 3 to 12 hours, preferably 5 to 6 hours. By-product water produced in this case is also distilled off. In this case, distillation of lactid is preferably avoided. However, since the reaction product contains almost no lactid, it is not required to specially lower the decompression rate.

Lactic acid oligomers produced by reaction in the above third heating step have an average polymerization degree of 3 to 30, preferably 3 to 23, and contain cyclic oligomer in the proportion of 90 weight % or more, preferably 99 weight % or more.

The above methods A, B and C merely show some of specific examples of methods of producing a mixture of poly lactic acids used in the present invention. A mixture of poly lactic acids which is produced by other methods can also be used in the present invention.

The dosage form of the agent of the present invention is not particularly limited, and any form suitable for the purpose of therapy or prevention can be selected from dosage forms for oral or parenteral administration. Examples of dosage forms suitable for oral administration include a tablet, a capsule, a powder, a granule, a parvule, a syrup, a solution, an emulsion, a suspension, a chewable tablet, and the like. Examples of dosage forms suitable for parenteral administration include, but are not limited to, transcutaneous absorbing agents in the form of an injection (e.g. a subcutaneous, intramuscular or intravenous injection, and the like), a drop, an inhalant, a nebula, a suppository, a gel, ointment or the like, and transcutaneous absorbing agents in the form of a trans-mucous absorbing agent, a patch agent, a tape agent or the like.

Liquid formulations suitable for oral administration such as a solution, emulsion or syrup can be produced using water, sugars such as sucrose, sorbit or fructose; glycols such as polyethylene glycol or propylene glycol; oils such as sesame oil, olive oil or soybean oil; antiseptics such as p-hydroxybenzoate; and flavors such as strawberry flavor and peppermint. In order to produce solid formulations such as capsule, tablet, powder or granule, there can be used an excipient such as lactose, glucose, sucrose or mannite; a disintegrator such as starch or sodium alginate; a lubricant such as magnesium stearate or talc; a binder such as polyvinyl alcohol, hydroxypropylcellulose or gelatin; a surfactant such as fatty acid ester; and a plasticizer such as glycerine, and the like.

Formulations for an injection or drop that are suitable for parenteral administration preferably comprise, as an active ingredient, the above substance in a dissolved or suspended state in a sterilized aqueous medium which is isotonic to the recipient's blood. For example, in the case of an injection, a solution can be prepared using an aqueous medium consisting of a saline solution, a glucose solution or a mixture of a saline solution and a glucose solution. In the case of a formulation for intestinal administration, it can be prepared using carriers such as theobroma oil, hydrogenated lipids or hydrogenated carboxylic acid, and can be provided as a suppository. In order to produce a nebula, the above substance as an active ingredient may be dispersed as microparticles, and a carrier which does not irritate the recipient's cavitas oris and respiratory tract mucosa and which facilitates absorption of the active ingredient can be used. Specific examples of carriers include lactose, glycerine, and the like. Formulations having a form such as aerosol or dry powder may be prepared depending on the properties of the substance of an active ingredient and the carrier to be used. One or two or more auxiliary ingredients selected from glycols, oils, flavors, an antiseptic, an excipient, a disintegrator, a lubricant, a binder, a surfactant, a plasticizer and the like may be added to these formulations for parenteral administration.

The dose and dosage frequency of the agent of the present invention are determined as appropriate, depending on various factors such as type and severity of the disease, dosage form, condition such as age or body weight of a patient, and presence or absence of complication. Generally, the dose of an active ingredient per day is 20 to 2,000 mg/kg, preferably 20 to 200 m/kg, and more preferably 50 to 150 mg/kg. It is preferred that the above dose of the agent is dividedly applied about once to 4 times per day, preferably about twice to 4 times per day. The agent of the present invention can be administered to any mammal including humans, and is preferably administered to human.

The time of administration of the agent of the present invention is not limited, and the agent can be administered at any time, including before or after surgery of cancer and before or after transplantation, but the administration is preferably started prior to surgery of cancer or transplantation. Especially in the case of using the agent of the present invention as an agent for preventing cancer recurrence after surgery, the effect of the present invention can be more effectively achieved by administering the agent prior to the surgery. Similarly, in the case of using the agent of the present invention as an agent for preventing the implantation and/or metastasis of cancer cells existing in the transplanted organ, the graft and/or the transplanted cells in a recipient, the effect of the present invention can be achieved by starting administering the agent prior to transplantation.

The mixture of cyclic and/or straight chain poly lactic acids having a condensation degree of 3 to 19 used in the present invention is used not only as an agent for preventing the implantation of cancer cells and/or an agent for preventing the cancer recurrence, but also may be mixed into a drinkable preparation, such as a nutrition supplement drinkable preparation, or can be mixed into food such as health food as a food additive. Specific examples of products of food and drink according to the present invention which comprises the mixture of cyclic and/or straight chain poly lactic acids having a condensation degree of 3 to 19, include health foods or supplements including drinks, such as those generally called a soft drink, drinkable preparation, health food, specified supplement food, functional food, function-activating food, nutritional supplementary food, supplement, feed, feed additive and the like.

Specific examples of food and drink into which the mixture of poly lactic acids can be mixed include confectionary, such as a chewing gum, chocolate, candy, sweet tablet, jelly, cookie, biscuit, and yogurt; frozen deserts, such as ice cream and sherbet; beverages, such as tea, soft drink (including juice, coffee, cocoa and the like), nutrition supplement drinkable preparation, and cosmetic drinkable preparation; and all other food and drink, such as bread, ham, soup, jam, spaghetti, and frozen food. Alternatively, the mixture of poly lactic acids used in the present invention can also be used by adding to seasoning, food additives, and the like.

By the use of the above food and drink of the present invention, there can be provided safe food and drink which can exert the effects of preventing the implantation of cancer cells and/or preventing the cancer recurrence and show substantially no toxic side effect.

The food and drink of the present invention encompasses food and drink in every form, and the types are not specifically limited. That is, the food and drink can be provided by mixing the mixture of cyclic and/or straight chain poly lactic acids having a condensation degree of 3 to 19 into the above-mentioned various food and drink, or various nutrient compositions, such as various oral or enteral nutrient preparations or drinks. Compositions of such food and drink may include protein, lipid, carbohydrate, vitamin and/or mineral, in addition to the mixture of cyclic and/or straight chain poly lactic acids having a condensation degree of 3 to 19. The form of the food and drink is not specifically limited, and may be in any form, such as solid, powdery, liquid, gel, and slurry forms, so far as it is in a form that is easily ingested.

The content of the mixture of poly lactic acids in the food and drink is not specifically limited, and is generally 0.1 to 20 weight %, more preferably approximately 0.1 to 10 weight %.

The mixture of poly lactic acids is preferably contained in the food and drink in an amount which achieve the object of the present invention. Preferably, about 0.1 g to 10 g, more preferably about 0.5 g to 3 g, of the mixture of poly lactic acids is contained per food or drink to be ingested.

The present invention is further described in the following examples, but the scope of the present invention is not limited by the examples in any way.

### EXAMPLES

### Production Example 1: Production of a mixture of poly lactic acids

500 ml of L-lactic acid (to which D-lactic acid was also mixed) was placed into a separable flask in a mantle heater. 300ml/min of nitrogen gas was flowed therein while stirring. Accumulated water was introduced into a flask equipped with a reflux condenser via a warmed descending type connecting tube, while heating at 145°C for 3 hours. Furthermore, after pressure was reduced to 150 mmHg and heated at the same temperature for 3 hours, the mixture was heated at 155°C for 3 hours under a reduced pressure of 3 mmHg, and then at 185°C for 1.5 hours under a reduced pressure of 3 mmHg to obtain poly lactic acids as a reaction product.

The obtained poly lactic acids were kept at 100°C, and 100ml of ethanol and 400ml of methanol were separately added thereto, and then the mixture was allowed to be cooled. This mixture was added to 500ml of methanol, and the mixture was well stirred and left to stand. Then, the mixture was filtrated for purification. The filtrate was subjected to vacuum drying and then dissolved in acetonitrile to obtain 200ml (stock solution) in total.

The stock solution was subjected to a reverse phase ODS column (TSK gel ODS-80 TM) which was previously equilibrated, and was stepwise eluted with 30%, 50% and 100% acetonitrile (pH2.0) each containing 0.01M hydrochloric acid to obtain poly lactic acids (condensation degree of 3 to 19) as an acetonitrile 100% elution fraction. The mass spectrum of the obtained substance is shown in Fig. 1. As is clear from the regular fragment ion peaks in Fig. 1, the obtained mixture of poly lactic acids mainly comprises cyclic condensate, and a small amount of linear condensate is contained therein.

### Test Example 1:

After giving the food mixed with the poly lactic acid mixture (those prepared in Production Example 1) at 10g/kg (1% by weight) or the usual food which does not contain a poly lactic acid mixture to mice of 4-week age and breeding for one month, Lewis lung cancer cells (1000 cells/mouse) were transplanted in the femoral muscle. The mice were divided into the individual group which was given with the usual food (control group : 11 animals), and the individual group which was given with poly lactic acid mixture containing food (CPL group : 11 animals).

As a result of continuing breeding after the transplanting of Lewis lung cancer cells (1000 cells/mouse), 8 animals among 11 animals of the control group showed carcinogenesis (rate of tumor implantation : 72.3%), and 5 animals among the 8 animals which showed carcinogenesis died within 30 days after the transplantation of cancer. On the other hand, 4 animals among 11 animals of the CPL group showed carcinogenesis (rate of tumor implantation : 36.4%), and 1 animal among the 4 animals which showed carcinogenesis died within 30 days.

Moreover, the averages of the tumor volumes of the cancer-bearing individuals in the control group and the CPL group at 30th day after transplanting (each group contains 3 individuals as surviving individuals) were 4850.9 mm³ and 4991.0 mm³ respectively. The tumor volume (1224.6 mm³) of one individual in the CPL group was clearly less than the other individuals.

Although the rate of implantation of a transplanted cancer cells was reduced approximately to half by administration of CPL as shown in the above result, there was no significant difference between the tumor volumes when carcinogenesis was shown. From these results, it is understood that the previous administration for 1 month before transplantation shows little effect on the inhibition of tumor growth under this condition, but is clearly effective for preventing implantation.

The tumor-bearing individuals (3 individuals in each group) was dissected on the 30th day after transplantation of cancer, and the size of the cancer colony on the surface of lung was classified in the following five grades for evaluating lung metastasis, and the number of colonies was measured. The obtained results are shown in Table 1.
5+: colony which spreads broadly over whole lung lobe
4+: colony of 2 to 3mm or more
3+: colony of approximately 1mm
2+: colony of less than 1mm
1+: minute colony

**Table 1**

| | 5+ | 4+ | 3+ | 2+ | 1+ |
|---|---|---|---|---|---|
| Control group | 2 | 10 | 16 | 18 | 36 |
| CPL group | 1 | 3 | 1 | 4 | 20 |

The colony which was evaluated as 5+ in the CPL group was an enormous cancer tissue which spreads over about 80% area of the whole lungs. Moreover, all individuals except one individual of the CPL group showed metastasis of cancer in paraaortic lymph node and mediastinal lymphnode.

### Industrial Utility

The agent and food and drink according to the present invention is useful for preventing the implantation of cancer cells and/or preventing the cancer recurrence, and can be used, for example, as an agent for preventing recurrence after surgery of cancer or for preventing the implantation and/or metastasis of cancer cells existing in the transplanted organ, the graft and/or the transplanted cells in a recipient. Further, the mixture of poly lactic acids used as an active ingredient in the present invention is a low condensate of lactic acids derived from organism components, and so it has a high biocompatibility with few side effects.

## Claims

1. An agent for preventing the implantation of cancer cells which comprises, as an active ingredient, a mixture of cyclic and/or straight chain poly lactic acids having a condensation degree of 3 to 19.

2. An agent for preventing the cancer recurrence which comprises, as an active ingredient, a mixture of cyclic and/or straight chain poly lactic acids having a condensation degree of 3 to 19.

3. The agent of claim 1 or 2 which is used for preventing the implantation and/or metastasis of cancer cells existing in the transplanted organ, the graft and/or the transplanted cells in a recipient.

4. The agent of claim 1 or 2, the administration of which starts prior to surgery of cancer

5. The agent of claim 3, the administration of which starts prior to transplantation.

6. The agent of any one of claims 1 to 5, wherein the lactic acid that is a repeating unit in the poly lactic acid consists substantially of L-lactic acid.

7. The agent of any one of claims 1 to 6, wherein the mixture of cyclic and/or straight chain poly lactic acids having a condensation degree of 3 to 19 is a fraction obtained by condensing lactic acids by dehydration under an inactive atmosphere, subjecting the ethanol- and methanol-soluble fractions of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

8. The agent of claim 7, wherein condensation by dehydration is performed by stepwise decompression and temperature rise under nitrogen gas atmosphere.

9. The agent of claim 7 or 8, wherein reverse phase column chromatography is performed by ODS column chromatography.

10. Food and drink for preventing the implantation of cancer cells and/or preventing the cancer recurrence, which comprises, as an active ingredient, a mixture of cyclic and/or straight chain poly lactic acids having a condensation degree of 3 to 19.
